# EUROPEAN PATENT APPLICATION

(11) **EP 3 492 030 A1**
(43) Date of publication of application: **05.06.2019**
(21) Application number: 17836261.2
(22) Date of filing: 10.07.2017
(51) Int. Cl.: A61B 17/34, A61B 17/00

(54) **PUNCTURE DEVICE SEALING MEMBRANE COMPRISING MULTI-DIMENSIONAL FLOATING FOLDS**

(30) Priority: 01.08.2016 CN 201610619956
(71) Applicant: 5R Med Technology (Chengdu) Co., Ltd, Chengdu, Sichuan 610000 (CN)
(72) Inventor: ZHU, Moshu, Chengdu Sichuan 610000 (CN)
(74) Representative: Gong, Jinping
(86) International application number: PCT/CN2017/092334
(87) International publication number: WO 2018/024079

(57) **Abstract**

The invention discloses a trocar seal membrane with multi-dimensional floating pleats. Said seal membrane comprises a proximal opening, a distal aperture, an outer seal body extending from the proximal opening to the distal aperture, and an inner seal body extending from the distal aperture to the proximal opening. Said outer seal body and said inner seal body extend to be intersected and form a flange; said distal aperture formed by a sealing lip for accommodating the inserted instrument and forming a gas-tight seal; said outer seal body includes at least one radial pleat; each radial pleat includes at least two annular-pleat-walls and at least one of the annular-pleat-walls include a plurality of tangential pleats. In the invention, under the essentially equivalent condition of geometric size, the deformation force of said multi-dimensional pleats is much smaller than the deformation force of said two-dimensional pleats

## Description

### FIELD OF THE INVENTION

The present invention relates to a minimally invasive surgical instrument, and in particular, to a trocar sealing element.

### BACKGROUD OF THE PRESENT INVENTION

A trocar is a surgical instrument, that is used to establish an artificial access in minimally invasive surgery (especially in rigid endoscopy). Trocars comprise in general a cannula and an obturator. The surgical use of trocars generally known as: first, make the initial skin incision at the trocar insertion site, then insert the obturator into the cannula, and then together they facilitated penetration of the abdominal wall through incision into the body cavity. Once penetrated into the body cavity, the obturator is removed, and the cannula will be left as access for the instrument to get in/out of the body cavity.

In rigid endoscopy surgery, it is usually necessary to establish and maintain a stable pneumoperitoneum for the sufficient surgical operation space. The cannula comprises a sleeve, an outer body, a seal membrane (also known as instrument seal) and a duck bill (also known as closure valve). Said cannula providing a channel for the instrumentation in/out of the body cavity, said outer body connecting the sleeve, the duck bill and the seal membrane into a sealing system; said duck bill normally not providing sealing for the inserted instrument, but automatically closing and forming a seal when the instrument is removed; said seal membrane accomplishing a gas-tight seal against the instrument when it is inserted.

In a typical endoscopic procedure, it is usually set up 4 trocars (access), i.e. 2 sets of small diameter cannula (normally 5 mm in diameter), and 2 sets of large diameter cannula (normally 10∼15 mm in diameter). Instruments, in general passing through a small cannula are only for ancillary works; herein one large cannula as an endoscope channel, and the other large cannula as the main channel for surgeon to perform surgical procedures. Through said main channel thereof, 5 mm diameter instruments used in approximately 80% of the procedure, and said large cannula used in approximately 20% of the procedure; furthermore, 5 mm instruments and large diameter instruments need to be switched frequently. The small instruments are mostly used, so that the sealing reliability of which is more important. The large instruments are more preferably used in a critical stage of surgery (Such as vascular closure and tissue suturing), therein switching convenience and operational comfort are more important.

Taking a 15 mm trocar in the clinical application (usually 15.8∼15.9 mm in diameter) as an example: when a 5 mm diameter instrument is used, it is approximately considered that the hoop force generated by the deformation of the sealing lip (i.e. the local material that forms the center hole of the seal membrane), ensures a reliable seal for the inserted instruments. There are a lot space for the 5 mm diameter instrument moves radially in the 15 mm diameter cannula (defining the longitudinal axis of the cannula to be the axial direction, defining the direction substantially perpendicular to said longitudinal axis to be the radial direction) . It is nevertheless favorable to operate the instruments from various extreme angles in surgery. Therefore, the seal membrane should be able to move radially, wherein the radial force on the seal membrane must be less than the radial component of said hoop force of the sealing lip, otherwise the sealing reliability will be damaged by said radial force when a 5 mm diameter instrument being used. It helps to maintain the sealing reliability by reducing the radial force of the seal membrane while moved radially, or reducing the bore diameter of the sealing lip to increase the hoop force of the sealing lip. However, reducing the bore diameter of the sealing lip inevitably leads to increase the hoop force of the sealing lip when the large-diameter instrument is inserted, which in turn leads to a large frictional resistance between the sealing membrane and the inserted instrument. Said large frictional resistance is normally easy to cause several defects, the seal membrane damaged or inverted, poor comfort of performance, fatigue performance, even the cannula insecurely fixed on the patient's abdominal wall etc., so the performance of trocar is affected. Therefore, a method of reducing the radial force is generally employed.

The seal membrane composed of an inner seal body and an outer floating pleats (or a bellows) is disclosed in US5407433, US5411483, US5792113, US5827228, US7789861, US7988671. Said outer floating pleats can reduce the radial force, thereby ensuring sealing reliability when a 5 mm diameter instrument is inserted. Although the shape or structure of the outer floating pleats in the prior art described above are different, there is a common feature that the outer floating pleats are formed by a waving line with pleats rotating around the axis of the center hole of the seal membrane. Create an arbitrary longitudinal section plane through said axis to intersect with the outer floating portion, the intersection line of which includes one or more radial pleats (or called transverse pleats); while make an arbitrary cross section plane perpendicular through said axis to intersect with the outer floating pleats, the formed intersection line of which is a cluster of concentric circles. Here defining this type of floating pleats as two-dimensional floating pleats.

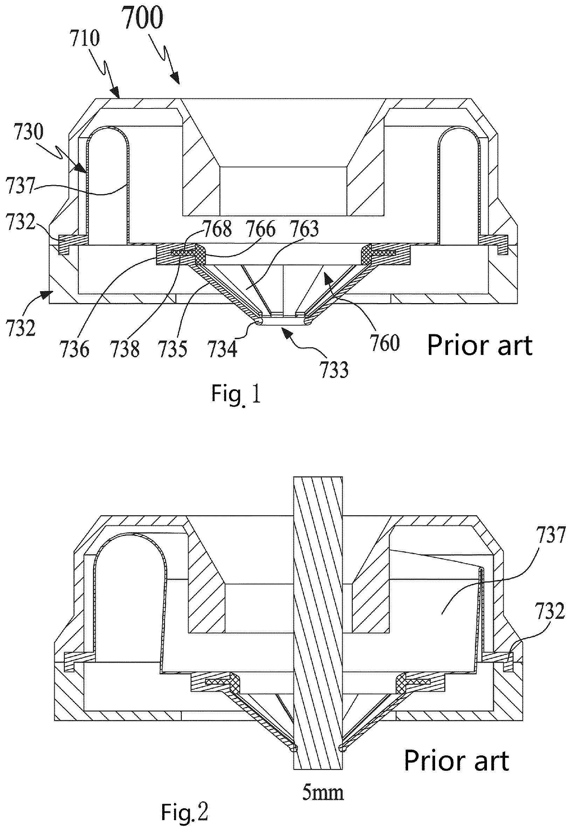

FIG. 1 depict a present typical seal assembly 700. Said seal assembly 700 comprises an upper housing 710, a lower housing 720, a seal membrane 730 and a protector 760. Said seal membrane 730 comprises a proximal opening 732, a distal aperture 733, a sealing lip 734, a sealing wall 735, a flange 736 and an outer floating portion 737. Said proximal opening 732 secured between the upper housing 710 and the lower housing 720; said floating portion 737 includes the radial pleats extending from the flange 736 to the proximal opening 732. In the present embodiment, comprising a radial pleat with two cylindrical pleated wall; said radial pleats allow the entire seal membrane floating radially in the seal housing formed by said upper housing 710 and said lower housing 720; said protector 760 comprising a plurality of mutually overlapping pieces 763, a wall 766, and a flange 768; said flange 768 is embedded in an inner groove 738 of said flange 736, so said protector 760 is fixed in the seal membrane 730.

FIG. 2 a simulated distorted view of said seal assembly 700 with the 5 mm diameter instrument inserted and moved to one side, and the deformation of the seal wall 735 is ignored in the figure. By theoretical analysis and refer to FIG. 2, when the seal membrane radially floating, it is necessary to force the two cylindrical pleated walls of the floating portion 737 to become egg-shaped; it means that it is necessary to force, as mentioned above, said all "concentric circles" have to be deformed into approximate ellipses, which will result in a larger radial force. In the prior art, the deformation force of said "concentric circles" is generally reduced by reducing the wall thickness of the floating pleats with ranging in size from 0.15 to 0.25 mm, such that leads to high precision requirements of the product mold which is complicated, and high rejection rate in the manufacturing process, thereby increasing production costs.

So far, there has not been disclosed a more effective method for reducing the radial force when the floating portion "concentric circles" is deformed.

### SUMMARY OF PRESENT INVENTION

In conclusion, one object of the invention is to provide a trocar seal membrane, said seal membrane comprising a proximal opening, a distal aperture, an outer seal body extending from the proximal opening to the distal aperture, and an inner seal body extending from the distal aperture to the proximal opening. Said seal membrane comprising a longitudinal axis and a transverse plane substantially perpendicular to said longitudinal axis, said distal aperture formed by a sealing lip for accommodating the inserted instrument and forming a gas-tight seal, said outer sealing body comprising both the radial pleats and the tangential pleats. Arbitrary longitudinal section plane through said longitude axis intersects the pleated portion of said outer seal body, the intersection line of which includes at least one radial pleat; arbitrary transverse section plane parallel to said transverse plane intersects the pleated portion of said outer seal body, the intersection lines of which are pleated-circuit, or one part of which is a pleated-circuit and the other parts are rings without pleats. It must be understood by those skilled in the art that when a small diameter instrument (for example a 5 mm diameter instrument) is inserted and moved radially (transversely) to an extreme position, said pleated-circuit compared to said concentric circles of the background: the deformation of said pleated-circuit mainly manifests as local bending deformation and displacement; and the deformation of said concentric circles is functioned as the entire circles are deformed into an egg shape; the deformation force of the pleated-circuit is smaller than which of concentric circles under the condition of the substantially equivalent geometrical size.

In the invention, one object is to provide a trocar integral seal membrane for minimally invasive surgery, said seal membrane comprising a proximal opening, a distal aperture, and an outer seal body extending from the proximal opening to the distal aperture, and an inner seal body extending from the distal aperture to the proximal opening; said outer seal body and said inner seal body extend to be intersected and form a flange; said distal aperture are formed by a sealing lip for accommodating the inserted instrument and forming a gas-tight seal; wherein said outer seal body includes at least one radial pleat; each radial pleat includes at least two annular pleated walls and at least one of the annular-pleat-walls include a plurality of tangential pleats.

In one aspect of the invention, therein, said annular-pleat-wall comprises an inner annular wall and an outer annular wall; said inner annular wall and/or said outer annular wall includes a plurality of tangential pleats that are substantially evenly distributed; said radial pleats and tangential pleats constitute multi-dimensional pleats, when the multi-dimensional pleats are moved radially, the deformation of the multi-dimensional pleats mainly is functioned as local bending deformation and displacement to reduce the deformation force.

In one aspect of the invention, the flange of the embodiment further includes an annular inner groove.

In another aspect of the invention, the cross-sectional profile of the radial pleats of the embodiment is V-shaped or U-shaped.

In another aspect of the invention, the cross-sectional profile of the tangential pleats of the embodiment is V-shaped or U-shaped.

In another aspect of the invention, the number of tangential pleats of the embodiment is 12.

In the invention, another object is to provide a split-type seal membrane for minimally invasive surgery. Said seal membrane comprises an inner seal body and an outer seal body, said inner seal body comprises the distal aperture, a first flange and an inner seal wall extending from the distal aperture to first flange; said outer seal body comprises the proximal opening, a second flange and an outer seal wall extending from the proximal opening to the second flange; wherein, said outer seal body includes at least one radial pleat; each radial pleat includes at least two annular-pleat-walls and at least one of the annular-pleat-walls with a plurality of tangential pleats; said first flange and said second flange are connected.

In the invention, another object is to provide an instrument seal assembly, wherein said instrument seal assembly comprises said seal membrane, a lower retainer ring, a upper retainer ring, a protect device, a upper body and a upper cover; said seal membrane and said protect device are sandwiched between the upper retainer ring and the lower retainer ring, while said proximal opening are sandwiched between the upper body and the upper cover.

In the invention, another object is to provide a trocar that comprises said instrument seal assembly, a sleeve, a duckbill seal and a lower housing; said duckbill seal secured between the sleeve and the lower housing is formed the first seal assembly, and said instrument seal assembly and the first seal assembly are secured together by a quick release device.

It is believed that the above invention or other objects, features and advantages, will be understood with the drawings and detailed description.

### DESCRIPTION OF THE DRAWINGS

A more complete appreciation of this invention, and many of the attendant advantages thereof will be readily apparent as the same becomes better understood by reference to the following detailed description, where:
FIG. 1 is a longitudinal sectional view of the seal assembly in the prior art;
FIG. 2 is a simulated distorted view of the seal assembly of FIG. 1 in the prior art with the 5 mm diameter instrument inserted and moved radially to an extreme position;
FIG. 3 is a 3D perspective partial sectional view of the cannula in the invention;
FIG. 4 is an exploded view of the seal membrane assembly in the cannula in FIG. 3;
FIG. 5 is a 3D perspective partial sectional view of the seal membrane assembly in FIG. 4;
FIG. 6 is a 3D perspective view of integral seal membrane in FIG. 4;
FIG. 7 is a sectional view along-line 7-7 in FIG. 6;
FIG. 8 is a sectional view along-line 8-8 in FIG. 6;
FIG. 9 is a sectional view along-line 9-9 in FIG. 8;
FIG. 10 is a 3D perspective partial sectional view of the cannula in the invention;
FIG. 11 is a 3D perspective partial sectional view of the seal membrane in FIG. 10;
FIG. 12 is a sectional view along-line 12-12 in FIG. 10;
FIG. 13 is a sectional view along-line 13-13 in FIG. 10;
FIG. 14 is a 3D perspective view of the seal membrane of the third embodiment according to the invention;
FIG. 15 is a sectional view along-line 15-15 in FIG. 14;
FIG. 16 is a sectional view along-line 16-16 in FIG. 14;
FIG. 17 is a sectional view along-line 17-17 in FIG. 16;
FIG. 18 is a 3D perspective view of the seal membrane of the forth embodiment according to the invention;
FIG. 19 is a sectional view along-line 19-19 in FIG. 18;
FIG. 20 is a sectional view along-line 20-20 in FIG. 18; and
FIG. 21 is a sectional view along-line 21-21 in FIG. 20;

In all views, the same referred number shows the same element or assembly.

### DETAILED DESCRIPTION OF PREFERRED EMBODIMENTS

Embodiments of the invention are disclosed herein, however, it should be understood that the disclosed embodiments are merely examples of the invention, which may be implemented in different ways. Therefore, the invention is not intended to be limited to the detail shown, rather, it is only considered as the basis of the claims and the basis for teaching those skilled in the art how to use the invention.

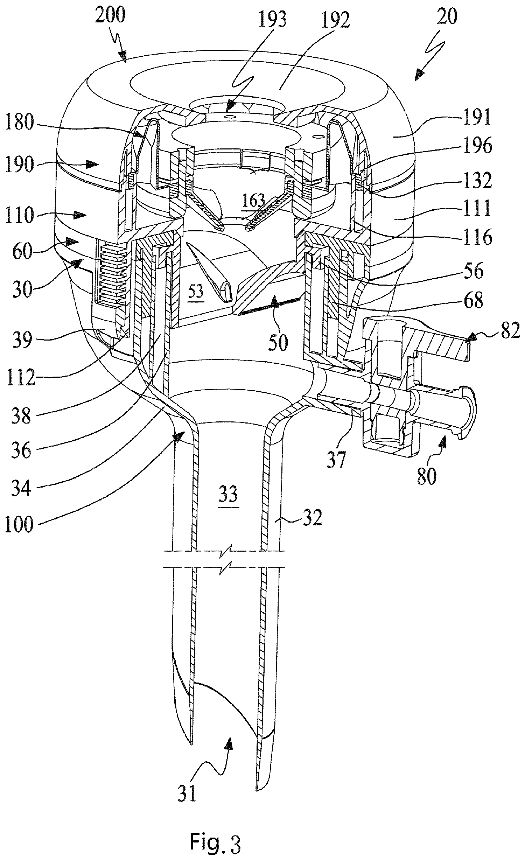

FIG. 3 shows an overall view of the trocar structure. Atypical trocar comprises an obturator 10 (not shown) and a cannula 20. The cannula 20 comprises an open proximal end 192 and an open distal end 31. In a typical embodiment, said obturator 10 passes through 10 said cannula assembly 20, together they facilitated penetration of the abdominal wall through incision into the body cavity. Once penetrated into the body cavity, the obturator 10 is removed, and the cannula 20 will be left as access for the instrument get in/out of the body cavity. Said proximal end 192 in the external position of the patient and said distal end 31 in the internal position. A preferred cannula 20 can be divided into the first seal assembly 100 and the second seal assembly 200. Locking receptacle 39 in said assembly 100 can be locked with snap-in projection 112 in said assembly 200. The cooperation of snap-in projection 112 and the locking receptacle 39 can be quickly released by one hand. The main purpose is for convenience of taking out tissues or foreign matter from the patient in the surgery. There are multiple ways to implement the quick release connection of said seal assembly 100 and assembly 200. In addition to the structure shown in this embodiment, a threaded connection, a rotary snap-in or other quick lock structure also may be applied. Alternatively, said assembly 100 and assembly 200 can be designed as a structure that cannot be split quickly.

FIG. 3 shows the composition and assembly relationship of the first seal assembly 100. The lower housing body 30 includes an elongated tube 32, which defines the sleeve 33 passed through the distal end 31 and is connected to the outer casing 34. Said lower housing body 30 comprises an inner wall 36 supporting duck bill seal and a valve bore 37 that communicates with the inner wall 36. The plunger 82 mounted in the valve body 80, the said two are mounted into said valve bore 37. The flange 56 of the duck bill seal 50 is sandwiched between the inner wall 36 and the lower cover. There are various ways of fixing between the lower cover 60 and the lower housing body 30, such as the interference fit, ultrasonic welding, glue bonding, and snap fastening. In this embodiment, 4 cylinders 68 of said lower cover 60, 4 holes 38 of said the lower body 30 are adopted to interference fit, so that the duckbill seal 50 is in the compressed state. Said tube 32, said the inner wall 36, said duck bill seal 50, said valve body 80 and said plunger 82 together are comprised the first chamber. Said duck bill seal 50, in this embodiment, is a single-slit, however, other types of closure valves may also be used, including flapper valves, multi-silted duck bill valves. When the instrument is passed through duck bill seal 50, the duckbill 53 will be opened, but it generally does not provide a complete seal against the instrument. When the instrument is removed, said duckbill 53 closed and substantially prevents insufflation fluid from escaping through the first chamber.

FIG. 3 shows the composition and assembly relationship of the second seal assembly 200. The seal membrane assembly 180 is sandwiched between the upper cover 110 and the upper body 190. The proximal end 132 of the seal membrane assembly 180 is secured between the inner ring 116 of the upper cover 110 and the inner ring 196 of the upper body 190. There are various secured ways between the upper body 190 and the upper body 110, such as the interference fit, ultrasonic welding, glue bonding, and snap fastening. The connection method, shown in this embodiment, is the outer shell 191 of the upper body 190 and the outer shell 111 of the upper cover 110 are secured by ultrasonic welding, so that the proximal end 132 of the seal membrane assembly 180 is in the compressed state. The central hole 113 of said upper cover 110, said inner ring 116, and said seal membrane assembly 180 together are comprised the second chamber.

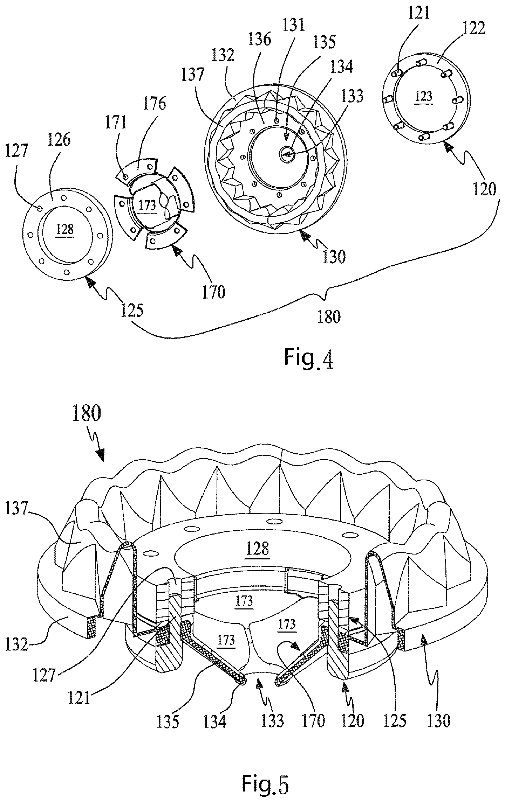

FIG. 3-4 show the composition and assembly relationship of the seal membrane assembly 180, which including a lower retainer ring 120, an upper retainer ring 15, the integral seal membrane 130 and a protection device 170. Said the seal membrane 130 and said protection device 170 are sandwiched between the lower retainer ring 120 and the upper retainer ring 125, moreover, the cylinder 121 of the said lower retainer ring 120 is aligned with corresponding holes on other components in said assembly 180. Said cylinder 121 and the bore 127 of the upper retainer ring are adopted to interference fit, so that the whole seal membrane assembly 180 in the compressed state. Said protection device 170 includes 4 protectors 173 arranged so as to protect a central sealing body of said seal membrane 130, herein permit the sharp edge of the instrument to pass through without causing perforations or tears to said seal membrane 130.

Said seal membrane 130 includes a proximal opening 132, a distal aperture 133, and a sealing wall extending from the proximal opening to the distal aperture 133. An outer floating portion 137 extends from said proximal opening 132 to the distal end 132; the inner seal body 135 extends from said distal aperture 133 to the proximal end; the outer floating portion 137 and the inner seal body 135 extend to be intersected at flange 136. Said distal aperture 133 formed by a sealing lip 134 for accommodating an inserted instrument and forming a gas-tight seal. Said flange 136 is used to mount a protector.

Said assembly 180 can be made from a variety of material with a range of different properties. For instance, said seal membrane 130 is made of a super elastic material such as silicone or polyisoprene; said protector device 170 is made of a semi-rigid thermoplastic elastomer; and said lower retainer ring 120 and said upper retainer ring 125 are made of a relatively hard rigid material such as polycarbonate.

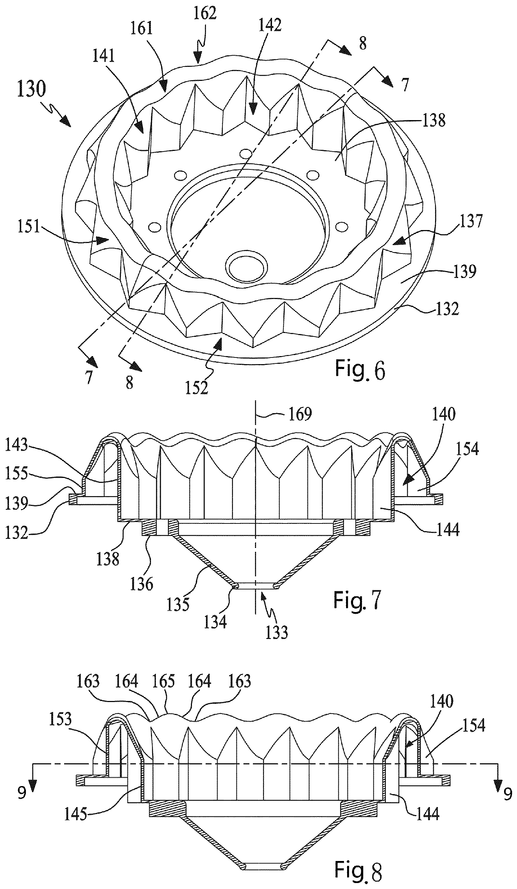

FIG. 6-9 show detailed depiction the seal membrane 130 of the first embodiment in the invention. Said outer floating portion 137 comprises the radial pleats 140 (or called transverse pleats 140), the wall portion 138 and the wall portion 139. One side of the wall portion 138 and said radial pleats 140 extend to be intersected, while the other side of which and the flange 136 extend to be intersected; One side of the wall portion 139 and said radial pleats 140 extend to be intersected, while the other side of which and the flange 136 extend to be intersected. Said proximal opening 132 comprising a central axis 169; make a longitudinal section plane through said axis 169 intersects said radial pleats 140, the shape of which intersection line is approximately U-shaped, which means said radial pleats 140 includes a U-shaped radial pleat. Said radial pleats 140 include an inner annular wall 141, an outer annular wall 151, and a top annular wall 161.

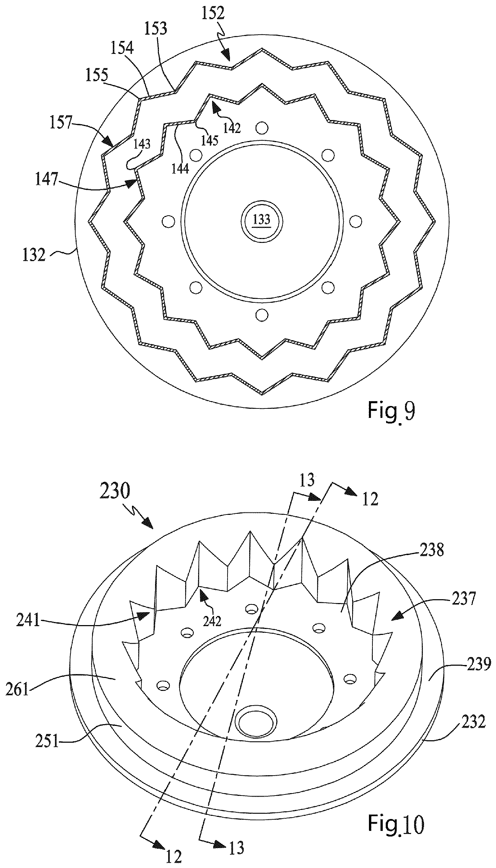

Said inner annular wall 141 includes a plurality of the tangential pleats 142 (or called circumferential pleats 142). There are 16 said tangential pleats 142, in the present embodiments, a more or less number of pleats may be employed. Each pleat 142 includes a pleated wall 144 which extends between the wave peak 145 and the wave trough 143. Said tangential pleats 142 are approximately evenly distributed on the inner annual wall 141 around said axis 169. Said outer annular wall 151 includes a plurality of the tangential pleats 152 (or called circumferential pleats 152). There are 16 said tangential pleats 152, in the present embodiment, a more or less number of pleats may be employed. Each pleat 152 includes a pleated wall 154 which extends between the wave peak 155 and the wave trough 153. Said tangential pleats 152 are approximately evenly distributed on the outer annual wall 151 around said axis 169. Said top annular wall 161 includes a plurality of the tangential pleats 162 (or called circumferential pleats 162). There are 16 said tangential pleats 162, in the present embodiment, although a more or less number of pleats may be employed. Each pleat 162 includes a pleated wall 164 which extends between the wave peak 165 and the wave trough 163. Said tangential pleats 162 are approximately evenly distributed on the top annual wall 161 around said axis 169. As FIG. 9 illustrates, a transverse section 168 (not shown) which is generally perpendicular to the axis 169 intersects the radial pleats 140, the formed intersection line of which is two laps of inner pleated-circuit 147 and outer pleated-circuit 157.

For convenience of description, such pleats consisting of the radial pleats (or transverse pleats) and the tangential pleats (or circumferential pleats) are referred to as multi-dimensional pleats (or three-dimensional pleats). It must be understood by those skilled in the art that when a small diameter instrument inserted (such as a 5 mm diameter instrument) and moved radially (laterally) to a certain extreme position, the deformation of said multi-dimensional pleats is compared to the deformation of the two-dimensional pleat described in the background: the deformation of said multi-dimensional pleats mainly manifests as local bending deformation and displacement; while the deformation of said two-dimensional pleats mainly force the whole pleats become egg-shaped; under the essentially equivalent condition of geometric size, the deformation force of said multi-dimensional pleats is much smaller than the deformation force of said two-dimensional pleats. Researches have shown that, taking a 15 mm trocar for an example, if a two-dimensional external floating pleat is used, the central aperture of the seal membrane should be small enough (usually 3.8∼4.0 mm in diameter) to ensure that the radial component force of the hoop force in the central hole of the seal membrane for the inserted 5 mm diameter instruments is greater than radial tension of pleated deformation at said extreme position, thereby ensuring its sealing reliability. If multi-dimensional pleats are used, the central aperture of the seal membrane can be relatively large (usually 4.4∼4.6 mm), so that it also ensure that the radial component force of the hoop force in the central hole of the seal membrane for the inserted 5 mm diameter instruments is greater than radial tension of pleated deformation at said extreme position, thereby ensuring its sealing reliability. While if the aperture diameter of the seal membrane is 3.8∼4.0 mm, when a large diameter instrument is inserted, for example using the 15.8 mm surgical stapling, the cylinder hoop strain of which is 295%∼316%. While if the aperture diameter of the seal membrane is 4.4∼4.6 mm, when a large diameter instrument is inserted, for example using the 15.8 mm surgical stapling, the cylinder hoop strain of which is 243%∼259%. Reducing the radial force when the outer floating pleats are deformed, the size(dimension) of the central aperture of the seal membrane can be increased, and then it can greatly reduce the cylinder hoop strain (stress) when large diameter instruments are applied, thereby reducing the frictional resistance described in the background.

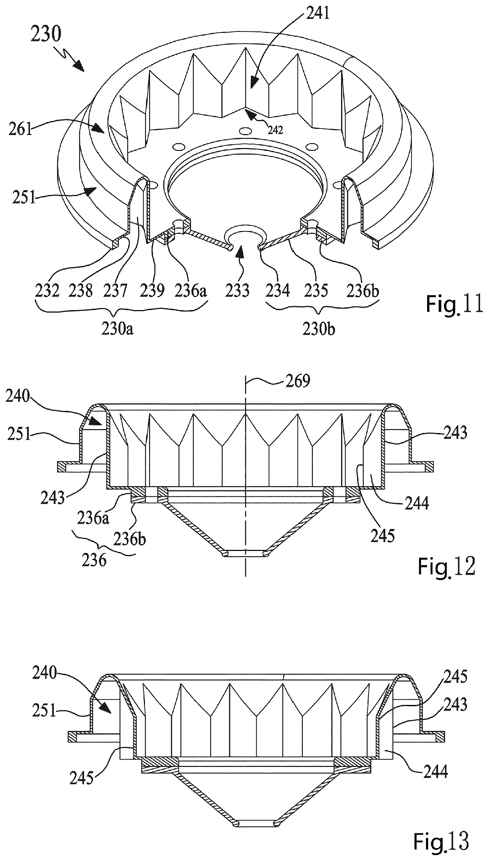

FIG. 10-13 show the split-type seal membrane 230 of the second embodiment in the present invention. The seal membrane 230 comprises an inner seal body 230b and an outer seal body 230a. Said inner seal body 230b includes a distal end aperture 233, the first flange 236b and an inner seal wall 235 which extends from the distal aperture 233 to the first flange 236b. Said outer seal body 230b includes a proximal opening 232, the second flange 236a and an outer seal wall 237 which extends from the proximal opening 232 to the second flange 236b. There are various manners for connecting said first flange 236b to said second flange 236b, such as which can be glued, welded, riveted or clamped, or in other mechanical fastening manners. For example, a seal membrane assembly comprising an outer seal body 230a, an inner seal body 230b, a protector, a lower retainer ring, an upper retainer ring. Said inner seal body 230b, said protect device 160 and said outer seal body 230a are sequentially stacked together, all of which sandwiched between the upper retainer ring and the lower retainer ring. Alternatively, the inner seal body 230b, the outer seal body 230a and the protection device 160are sequentially stacked together, all of which sandwiched between the upper retainer ring and the lower retainer ring. Those skilled in the art readily conceive that a gasket or gasket may be added between said inner seal body 230b and said outer seal body 230a. The use of a plit-type seal membrane can reduce the manufacturing difficulty of the inner seal and the outer seal, and improve the manufacturing precision of the product.

The outer sealing wall 237 comprises radial pleats 240 (or transverse pleats 240), the wall portion 238 and the wall portion 239. One side of the wall portion 238 and said radial pleats 240 extend to be intersected, while the other side of which and the second flange 236a extend to be intersected; One side of the wall portion 239 and said radial pleats 240 extend to be intersected, while the other side of which and said proximal opening 136 extend to be intersected. Said proximal opening 232 comprising a central axis 269, Make a longitudinal section plane through the axis 269 to intersect the radial pleats 240, the shape of which intersection line is approximately U-shaped, which means the radial pleats 240 includes a U-shaped radial pleat. Said radial pleat 240 includes an inner annular wall 241, an outer annular wall 251 and a top annular wall 261.

Said inner annular wall 241 includes a plurality of the tangential pleats 242 (or called circumferential pleats 242). There are 16 said tangential pleats 242, in the present embodiment, although a more or less number of pleats can be employed. Each pleat 242 includes a pleated wall 244 which extends between the wave peak 245 and the wave trough 243. Said tangential pleats 242 are approximately evenly distributed on the inner annular wall 241 around said axis 269.

As described in the first embodiment above, such pleats consisting of radial pleats (or transverse pleats) and tangential pleats (or called circumferential pleats) are referred to as multi-dimensional pleats (or three-dimensional pleats). It must be understood by those skilled in the art that, in the second embodiment, compared with the first embodiment, the effect of reducing the pleats deformation force during radially moving is not as good as that in the first embodiment.

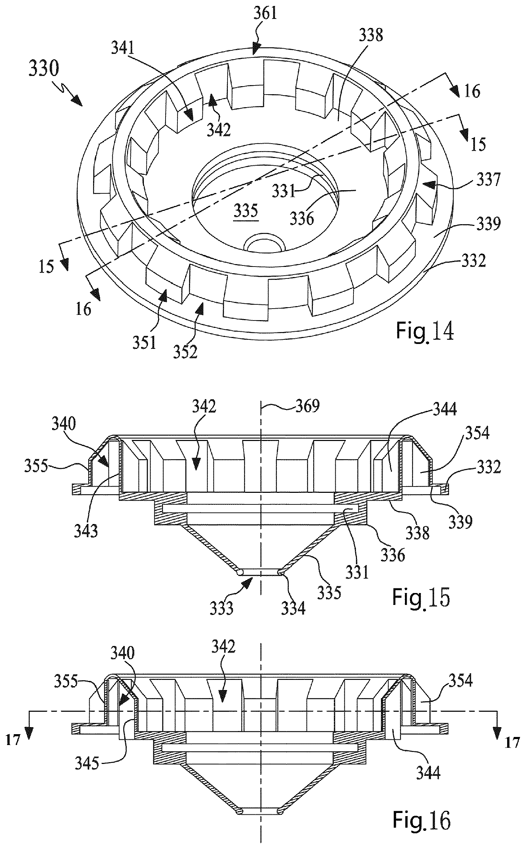

FIG. 14-17 show the seal membrane 330 of the third embodiment in the present invention. Said seal membrane 330 includes a proximal opening 332, a distal aperture 333, and the sealing wall extending from the proximal opening 332 to the distal aperture 333. An outer floating portion 337 extends from said proximal opening 332 to the distal end; the inner seal body 335 extends from said distal aperture 333 to the proximal end; the outer floating portion 337 and the inner seal body 335 extend to be intersected at flange 336. Said distal aperture 333 formed by a sealing lip 334 for accommodating an inserted instrument and forming a gas-tight seal. Said flange 336 includes an annular inner groove 331, which is used to mount a protection device 760.

Said outer folding portion 337 comprises the radial pleats 340(or called transverse pleats), the wall portion 338 and the wall portion 339. One side of the wall portion 338 and said radial pleats 340 extend to be intersected, while the other side of which and the flange 336 extend to be intersected; One side of the wall portion 339 and said radial pleats 340 extend to be intersected, while the other side of which and the proximal opening 332 extend to be intersected. Said proximal opening 332 comprising a central axis 369. Make a longitudinal section plane through said axis 369 intersects said radial pleats 340, the shape of which intersection line is approximately U-shaped, which means said radial pleats 340 includes a U-shaped radial pleat. Said radial pleats 340 include an inner annular wall 341, an outer annular wall 351, and a top annular wall 361.

Said inner annular wall 341 includes a plurality of the tangential pleats 342 (or called circumferential pleats 342). In the present embodiment there are 12 said tangential pleats 342, yet a more or less number of pleats can be employed. Each pleat 342 includes a pleat side wall 344 which extends between the pleat top wall 345 and the pleat bottom wall 343. Said tangential pleats 342 are approximately evenly distributed on the inner annular wall 341 around said axis 369. Said outer annular wall 351 includes a plurality of the tangential pleats 142(or called circumferential pleats 352). In the present embodiment, there are 12 said tangential pleats 352, a more or less number of pleats may be employed. Each pleat 352 includes a pleat side wall 354 which extends between the pleat top wall 355 and the pleat bottom wall 353. Said tangential pleats 352 are approximately evenly distributed on the outer annual wall 351 around said axis 369. As FIG. 16 illustrates, a transverse section 368 (not shown) which is generally perpendicular to the axis 369 intersects the radial pleats 340, and the formed intersection line of which is two laps of inner pleated-circuit 347 and outer pleated-circuit 357. The cross-sectional profile of said tangential pleats 142 and said tangential pleats 152 in the first embodiment is approximately V-shaped. While the cross-sectional profile of said tangential pleats 342 and said tangential pleats 352 in the third embodiment is approximately U-shaped; the effect of the third embodiment is similar to the first embodiment in reducing the radial force when the outer floating portion is radially moved to the extreme position.

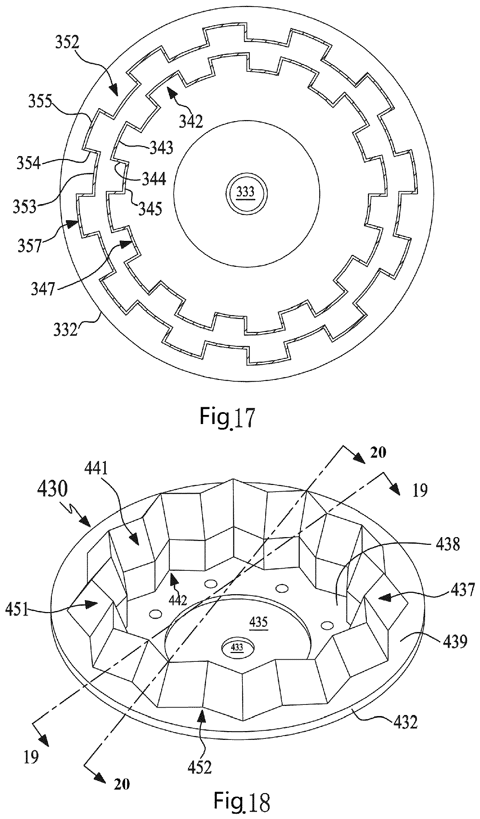

FIG. 18-21 show the seal membrane 430 of the forth embodiment in the present invention. Said seal membrane 430 includes the proximal opening 432, the distal aperture 433, and the sealing wall extending from the proximal opening 432 to the distal aperture 433. An outer floating portion 437 extends from said proximal opening 432 to the distal end; the inner seal body 435 extends from said distal aperture 433 to the proximal end; the outer floating portion 437 and the inner seal body 435 extend to be intersected at flange 436. Said distal aperture 433 formed by a sealing lip 434 for accommodating an inserted instrument and forming a gas-tight seal. Said flange 436 is used to mount a protection device.

Said outer folding portion 437 comprises the radial pleats 440 (or called transverse pleats), the wall portion 438 and the wall portion 439. One side of side wall portion 438 and said radial pleats 440 extend to be intersected, while the other side of which and the flange 436 extend to be intersected; One side of the wall portion 439 and said radial pleats 440 extend to be intersected, while the other side of which and the proximal opening 432 extend to be intersected. Said proximal opening 432 comprising a central axis 469. A longitudinal section plane through said axis 469 intersects said radial pleats 440, the shape of which intersection line is approximately V-shaped, which means the radial pleats 440 includes a V-shaped radial pleat. Said radial pleats 440 includes an inner annular wall 441, an outer annular wall 451.

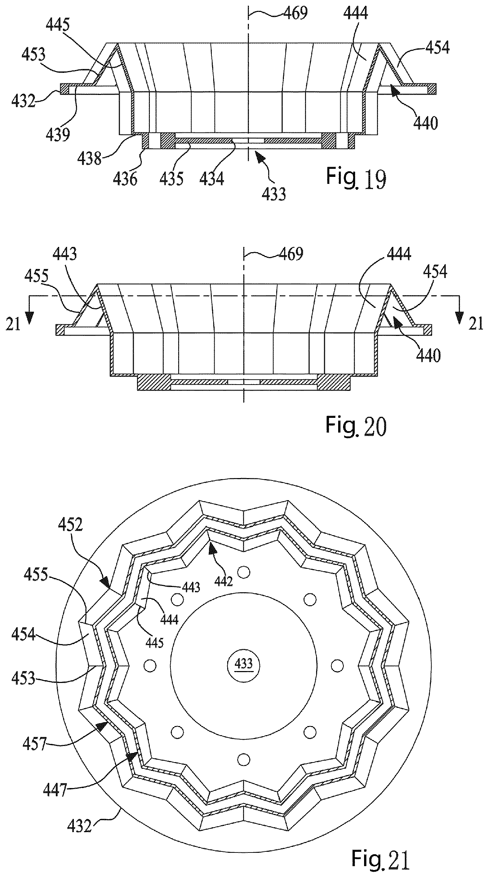

Said inner annular wall 441 includes a plurality of the tangential pleats 442(or called circumferential pleats 442). In the present embodiment, there are 12 said tangential pleats 442, yet a more or less number of pleats can be employed. Each pleat 442 includes a pleat side wall 444 which extends between the wave peak 445 and the wave trough 443. Said tangential pleats 442 are approximately evenly distributed on the inner annular wall 441 around said axis 469. Said outer annular wall 451 includes a plurality of the tangential pleats 142(or called circumferential pleats 452). There are 12 said tangential pleats 452, in the present embodiment, although a more or less number of pleats may be employed. Each pleat 452 includes a pleat side wall 454 which extends between the wave peak 455 and the wave trough 453. Said tangential pleats 452 are approximately evenly distributed on the outer annual wall 451 around said axis 469. As FIG. 20 illustrates, a transverse section 468 (not shown) which is generally perpendicular to said axis 469 intersects said radial pleats 440, the formed intersection line of which is two laps of inner pleated-circuit 347 and outer pleated-circuit 357.

The cross-sectional profile of said radial pleats 140 in the first embodiment is approximately U-shaped. While the cross-sectional profile of said radial pleats 440 in the fourth embodiment is approximately V-shaped; the effect of the forth embodiment is similar to the first embodiment in reducing the radial force when the outer floating portion is radially moved to the extreme position.

It will be readily apparent to those skilled in the art that a reasonable fillet transition can avoid stress concentration or easier deformation of certain areas. Since the diameter of the seal membrane is small, especially the diameter of the area near the sealing lip is smaller, such a small diameter and different chamfers that the appearance of the seal membrane looks different. In order to clearly show the geometric relationship of elements, the embodiment of the description in the invention is generally the graphics after removing fillet.

It would be comprehended that the shape of said radial pleats is approximately U-shaped, may also be approximately V-shaped, approximately triangular or arbitrary open polygon. In the present embodiment, said radial pleats contains only one pleat, however multiple radial pleats may be employed. The cross-sectional profile of said tangential pleats may be U-shaped, approximately V-shaped or a portion of the tangential pleats is U-shaped and the other portion of which is V-shaped. The approximate U-shape and the approximate V-shape described in this embodiment cannot be limited to the shape that must be U-shaped or V-shaped.

Many different embodiments and examples of the invention have been shown and described. One of those ordinary skilled in the art will be able to make adaptations to the methods and apparatus by appropriate modifications without departing from the scope of the invention. Such as, the structure and the manner of fixing of the protector disclosed in US7788861 are used in the example of the present invention. However, the structure and the manner of fixing of the protector disclosed in US7798671 can be used, and in some applications, the protector structure may not be included. In the invention, the positional relationship of the intersecting surfaces composed of said groove and the intersection line thereof are described with reference to specific embodiments, and the methods of increasing curved surfaces to form a multifaceted mosaic or using of the high-order curved surface to make the intersection line and the groove shape to look different from said embodiment. However, it can be considered not deviated from the scope of the invention, as long as it conforms to the general idea of the invention. Several modifications have been mentioned, to those skilled in the art, other modifications are also conceivable. Therefore, the scope of the invention should follow the additional claims, and at the same time, it should not be understood that it is limited by the specification of the structure, material or behavior illustrated and documented in the description and drawings.

## Claims

1. A trocar integral seal membrane for minimally invasive surgery, comprises a proximal opening, a distal aperture ,an outer seal body extending from the proximal opening to the distal aperture, and an inner seal body extending from the distal aperture to the proximal opening; said outer seal body and said inner seal body extend to be intersected and form a flange; said distal aperture formed by a sealing lip for accommodating the inserted instrument and forming a gas-tight seal; therein, said outer seal body includes at least one radial pleat; each radial pleat includes at least two annular pleated walls and at least one of the annular pleated walls with a plurality of tangential pleats.

2. Said seal membrane as defined in claim 1, wherein: said annular pleated wall includes the inner annular wall and the outer annular wall; said inner annular wall and/or said outer annular wall includes a plurality of the tangential pleats substantially evenly distributed; said radial pleats and tangential pleats constitute multi-dimensional pleats, when the multi-dimensional pleats are moved radially,the deformation of the multi-dimensional pleats mainly manifests as local bending deformation and displacement to reduce the deformation force.

3. Said seal membrane as defined in claim 1, wherein said flange including said annular inner groove.

4. Said seal membrane as defined in claim 1, wherein the cross-sectional shape of said radial pleats is V-shaped or U-shaped.

5. Said seal membrane as defined in claim 1, wherein the cross-sectional shape of said tangential pleats is V-shaped or U-shaped, or one portion of which is V-shaped and the other portion is U-shaped.

6. Said seal membrane as defined in claim 5, wherein the number of said radial pleats is 12.

7. A split-type seal membrane for minimally invasive surgery, comprises said inner seal body and said outer seal body,said inner seal bodyincluding the distal aperture, the first flange and the inner sealing wall which extends from sidedistal aperture to said first flange;said outer seal body including a proximal opening, the second flange and the outer seal wall which extends from the proximal opening to the second flange;wherein,said outer seal bodyincluding at least one radial pleat;each radial pleat including at least two annular pleated walls and at least one of the annular pleated walls includes a plurality of tangential pleats;the first flange and the second flange connected.

8. Said seal membrane as defined in claim 7, wherein said annular pleated wall includes the inner annular wall and the outer annular wall;said inner annular wall and/or said outer annular wall includes a plurality of the tangential pleats substantially evenly distributed;said radial pleats and tangential pleats constitute multi-dimensional pleats,when the multi-dimensional pleats are moved radially,the deformation of the multi-dimensional pleats mainly manifests as local bending deformation and displacement to reduce the deformation force.

9. A instrument seal assembly as defined in claims 1 to 8, wherein said instrument seal assembly comprises any one of said seal membranes, said instrument seal assembly including a lower retainer ring, a upper retainer ring, a protection device,a upper body and a upper cover;said seal membrane and said protector are sandwiched between the upper retainer ring and the lower retainer ring, while said proximal opening are sandwiched between the upper housing and the upper cover.

10. A trocar including said instrument seal assembly as defined in claim 9, wherein the trocarcomprises a sleeve, a duckbill seal and a lower housing, said duckbill seal secured between the sleeve and the lower housing forming the first seal assembly, said instrument seal assembly and the first seal assembly secured together by a quick release device.
